# EUROPEAN PATENT APPLICATION

(11) **EP 4 528 260 A1**
(43) Date of publication of application: **26.03.2025**
(21) Application number: 23807029.6
(22) Date of filing: 18.05.2023
(51) Int. Cl.: G01N 27/327, G01B 5/00, G01N 27/26

(54) **BIOSENSOR AND METHOD FOR PREPARING BIOSENSOR**

(30) Priority: 18.05.2022 CN 202210547593
(71) Applicant: Leadway (HK) Limited, Sheung Wan, Hong Kong (CN)
(72) Inventor: JI, Xubo, Hangzhou, Zhejiang 310030 (CN); LEI, Tianran, Hangzhou, Zhejiang 310030 (CN); WANG, Yi, Hangzhou, Zhejiang 310030 (CN); WU, Mengqun, Hangzhou, Zhejiang 310030 (CN); GUO, Tao, Hangzhou, Zhejiang 310030 (CN); ZHANG, Li, Hangzhou, Zhejiang 310030 (CN)
(74) Representative: Schiweck Weinzierl Koch Patentanwälte Partnerschaft mbB
(86) International application number: PCT/CN2023/095023
(87) International publication number: WO 2023/222078

(57) **Abstract**

The present invention provides a biosensor and a method for preparing the biosensor. The biosensor includes an insulating substrate, a sample injection port, a sample injection channel for entrance of a test sample, and a conductive layer disposed on the insulating substrate, engraved lines and electrodes formed through division by the engraved lines being distributed on the conductive layer, a reagent layer being disposed on part or all of the electrodes located in the region of the sample injection channel, and an auxiliary region being also disposed on the conductive layer under the other end of the sample injection channel opposite to the sample injection port. According to the present invention, by providing the auxiliary region on the conductive layer, the testing accuracy and stability of the biosensor are effectively ensured.

## Description

### Field of the Invention

The present invention relates to the field of biomedical testing, in particular to a biosensor for testing and a preparation method thereof.

### Background of the Invention

Electrochemical sensors for detecting the content of an analyte in a sample and their matched detectors have been widely used in the daily monitoring of diseases. For example, diabetic patients commonly use electrochemical sensors to monitor daily glucose concentrations in the blood.

The basic structure of such electrochemical sensors includes an electrode system disposed on an insulating substrate, the electrode system includes a plurality of or multiple types of electrodes such as a working electrode and a counter electrode, and a reaction reagent that reacts with the analyte covers the corresponding electrodes. A sample interlayer having a groove is located on the electrode, a cover plate with an air hole covers the sample interlayer, the insulating substrate, the interlayer and the cover plate form a sample injection channel, and the other end of the electrode system is provided with a contact contacting a contact pin of a detector. The sample flowing into the sample injection channel reacts with a reaction reagent on the electrodes to generate electrical signals from which the detector derives the test result.

The screen printing technology is utilized to print conductive materials on the insulating substrate to form electrodes, electrode connecting contacts and leads, which is a method commonly used for preparing electrochemical sensors at present. When electrochemical sensors are manufactured using the screen printing method, large batch-to-batch variations exist in aspects of certain parameters from batch to batch. In order to eliminate the extent to which the batch-to-batch variations affect the test results, the problem of batch-to-batch variations is generally solved by inserting a calibration chip into the detector to correct the test results. However, inserting a calibration chip not only increases the operation steps of an operator, but also leads to inaccurate test results if the operator forgets to insert or incorrectly inserts the calibration chip. Some researchers also adopted, for example, the method in Chinese Patent ZL201210095096.5 to correct the batch-to-batch variations by setting calibration messages on the sensor.

In order to solve the problem of cumbersome testing steps or complicated preparation process mentioned above, Chinese Patent ZL00803756.6 provides a method for forming a thin film electrode, i.e., a thin film-like conductor layer is uniformly spread on an insulating substrate in advance, and then the electrode is formed by a laser etching method. This method has relatively high manufacturing precision and basically can ensure no batch-to-batch variations between products produced in different batches, or that the presence of batch-to-batch variations will not affect the test results in the case where no correction is required.

On the conductive layer of this type of sensors, as the separation of different electrodes or conductive blocks of some use only relies on engraved lines obtained by the laser etching method, different electrodes or electrodes and adjacent conductive blocks thereof should be completely separated from each other to form an open-circuit state. Similarly, in order to ensure the accuracy of the test results, the electrode area reacting with the reagent layer in the open groove of the interlayer should be consistent from batch to batch of sensors and should not change due to the change of the relative position of the interlayer or the reagent layer.

Considering that the position of the interlayer or the reagent layer in the actual production process will inevitably deviate due to fluctuations in the process, the present patent is completed on the basis of the above demands, and the objective thereof is to provide a simple design which can ensure that the electrode area reacting with the reagent layer in the open groove of the interlayer will not change, so as to achieve the stability of the sensor performance.

### Summary of the Invention

The present invention is completed based on basic requirements of the sensor, and has an objective of providing a simple design which can ensure that the electrode area in the sample injection channel reacting with the reagent layer does not change.

The biosensor described in the present invention contains an insulating substrate and a conductive layer disposed on the insulating substrate, engraved lines being distributed on the conductive layer, and electrodes being formed on the conductive layer divided by the engraved lines. A test reagent is allocated to all or part of the electrodes at a sample contacting end. The biosensor further comprises a sample injection channel for entrance of the solution of a substance to be tested. The electrode extends from the sample contacting end to a contact end of the sensor, a test instrument generates an electrical feedback signal by applying an electrical signal to the electrodes or generates an electrical feedback signal by applying an electrical signal to promote the reaction of the substance to be tested with the reagent on the electrodes, and the electrical feedback signal is conducted back to the test instrument via the electrodes to achieve quantitative testing. In the process of conducting the electrical signal generated by the reaction of the substance to be tested with the reagent or other electrical signal required in the test process of the sensor to the contact end of the sensor, large deviation of the electrode areas that can react with the reagent layer in the sample injection channels of different sensors will lead to fluctuations of the electrical signal and thus generate deviation of the test results. In order to avoid the change of effective electrode areas caused by position deviations generated from process fluctuations of the interlayer or the reagent layer, an auxiliary region enclosed by an engraved line is disposed on the conductive layer at the most distal end of the open groove of the interlayer of the sensor or at the most distal end of the sample injection channel. By providing such an auxiliary region, it simply and effectively ensures the consistency of the effective electrode area within the sample injection channels of the biosensors of the same type and different batches, thus ensuring the accuracy of the test results of the biosensors.

In the biosensor of the first aspect of the present invention, all of the electrodes in the open groove of the interlayer, that will come into contact with the sample to be tested, are covered with a reagent layer, and at this point, the auxiliary region located at the most distal end of the open groove of the interlayer is at least partially exposed in the open groove, in other words, at this point, the lower edge of the open groove of the interlayer is on the top of, or flush with the lower edge of the auxiliary region, so as to achieve that the all electrode areas reacting with the reagent layer in the open groove of the interlayer of different biosensors of the same type reacting with the reagent layer is kept consistent.

In the biosensor of the second aspect of the present invention, all of the partial electrodes in the open groove of the interlayer that will come into contact with the sample to be tested are covered with the reagent layer. At this time, the auxiliary region located at the most distal end of the open groove of the interlayer is at least partially covered by the reagent layer, in other words, the lower edge of the reagent layer is on top of, or flush with, the lower edge of the auxiliary region, so as to achieve that all electrode area in the open groove of the interlayer reacting with the reagent layer is kept consistent.

The shape and size of the above auxiliary region can be set according to the actual situation.

The above aspects are further illustrated.

The present invention provides a biosensor, comprising an insulating substrate, a sample injection port, a sample injection channel for entrance of a test sample, and a conductive layer disposed on the insulating substrate, engraved lines and electrodes divided by the engraved lines being distributed on the conductive layer, a reagent layer being disposed on part or all of the electrodes located in the region of the sample injection channel, wherein an auxiliary region enclosed by an engraved line or by an engraved line and an edge line of the biosensor is disposed on the conductive layer under the other end of the sample injection channel opposite to the sample injection port, the transverse width of the auxiliary is greater than or equal to the width of the sample injection channel, and the projection of the lower edge of the sample injection channel on the conductive layer falls within the auxiliary region or on the border of the auxiliary region.

Preferably, the auxiliary region is located at the most distal end of the sample injection channel.

Further, the auxiliary region is further away from the sample injection port than the electrodes.

Further, the electrodes comprise a working electrode and a counter electrode, and the auxiliary region is further away from the sample injection port than the working electrode and the counter electrode; or the electrodes comprise a working electrode, a counter electrode and a reference electrode, and the auxiliary region is further away from the sample injection port than the working electrode, the counter electrode and the reference electrode.

Further, a part of the auxiliary region is located within the sample injection channel. Further, the lower edge of the reagent layer in the sample injection channel is located within auxiliary region.

The present invention provides a biosensor, comprising an insulating substrate and a conductive layer disposed on the insulating substrate, the conductive layer is divided by engraved lines to form electrodes, and an auxiliary region is disposed at least at the most distal end of the interlayer.

The present invention provides a method for preparing a biosensor, comprising the following steps: on an original material having an insulating substrate and a conductive layer, etching the conductive layer to form engraved lines and electrodes divided by the engraved lines; adding a prepared reagent to corresponding electrodes; and sticking an interlayer and an upper cover in sequence on the electrodes, wherein an auxiliary region enclosed by an engraved line or by an engraved line and an edge line of the biosensor is disposed on the conductive layer under the other end of the sample injection channel opposite to the sample injection port, the transverse width of the auxiliary is greater than or equal to the width of the sample injection channel, and the projection of the lower edge of the sample injection channel on the conductive layer falls within the auxiliary region or on the border of the auxiliary region.

Further, the auxiliary region is located at the most distal end of the sample injection channel.

Further, the auxiliary region is further away from the sample injection port than the electrodes.

Further, the etching method is laser cutting or laser marking.

Further, the specific steps include: step 1: according to a pre-designed electrode pattern, performing laser etching on an original material having an insulating substrate and a conductive layer to etch the electrode pattern; step 2: adding a test reagent to corresponding electrodes requiring addition; step 3: affixing an interlayer and an upper cover on the electrodes, and forming a sample injection channel at the sample injection end of the biosensor to obtain a semi-finished large card; and step 4: cutting the semi-finished large card by using a cutter along a preset cutting line to obtain finished biosensors used alone.

The engraved line that can form various patterns is etched on the conductive layer of the biosensor in step 1. An electrode pattern is etched using laser etching. The engraved lines are formed after etching. The conductive layer is selected from a gold film, a palladium film, an alloy film, a carbon film and other non-metallic conductive layer or a mixture thereof. The width of the engraved lines is within the range of 0.020mm to 0.200mm.

The present invention has the following beneficial effects: by providing a certain auxiliary region on the conductive layer, the present invention can ensure that the size of the effective electrode area will not be affected when the biosensor has inevitable position deviation in the preparation process, ensuring the testing accuracy and stability of the prepared biosensor.

### Brief Description of the Drawings

Fig. 1 is an exploded view of a biosensor of the solution of Example 1.
Fig. 2 is a schematic diagram of the distribution of an electrode system and engraved lines on a conductive layer corresponding to the biosensor in the solution of Example 1.
Fig. 3 is a flow chart of a manufacturing method of the biosensor.
Fig. 4 is a schematic diagram of multi-lined sensor base units A formed by laser engraving of the conductive layer of the biosensor.
Fig. 5 is a schematic diagram of the single-lined sensor base units A formed after cutting by a cutter along the cutting line 50 as shown in Fig. 4.
a, b and c in Fig. 6 are three positions of an interlayer on the biosensor, and different distribution situations of a reagent on an electrode 4.
Fig. 7 is a biosensor provided with an auxiliary region, a, b and c are three positions of the interlayer on the biosensor, and different distribution situations of a reagent on the electrodes.
Fig. 8 is a schematic diagram of different design shapes of the auxiliary region.
Fig. 9 is the situation when the reagent does not spread over the sample injection channel, and a, b, and c represent three different distribution situations of the reagent.
Fig. 10 is a biosensor provided with an auxiliary region, and a, b and c represent three different distribution situations of the reagent.
Fig. 11 is three different shape designs of the auxiliary region and the distribution situations of the reagent.
Fig. 12 is a biosensor provided with an auxiliary region enclosed by engraved lines and an edge line of the conductive layer.
Fig. 13 is a biosensor with the electrode 4 in linear arrangement and containing an auxiliary region.
Fig. 14 is a biosensor provided with an auxiliary region enclosed by an engraved line and a side edge line of the conductive layer.

### Detailed Description of the Embodiments

Hereinafter, the embodiment of the present invention is described with reference to the accompanying drawings, and furthermore, the embodiment shown here is merely an instance, and the present invention is not necessarily limited to this embodiment.

### Example 1:

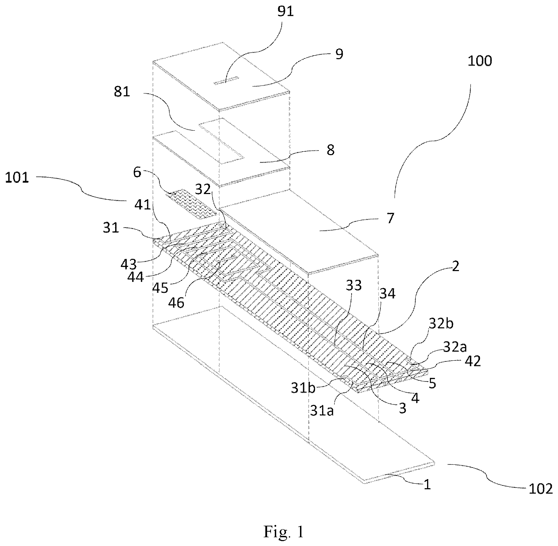

The biosensor 100 as shown in Fig. 1, Fig. 2 and Fig. 7 includes an insulating substrate 1, a conductive layer 2 disposed on the substrate, and an electrode system formed on the conductive layer through division by engraved lines 31, 31a, 31b, 32, 32a, 32b, 33, 34, 41, 42, 43, 44, 45, 46. The electrode system includes a working electrode 5, a counter electrode 4 and a reference electrode 3, and the electrodes have a sample contacting end 101 contacting a sample and a contact end 102 contacting a contact pin of an analyzer. A reagent layer 6 is added to corresponding electrodes at the sample contacting end. An interlayer 8 with an open groove 81 covers the sample contacting end of the electrodes, and an upper cover 9 covers the interlayer 8, so that the conductive layer 2, the open groove 81 and the upper cover 9 form a sample injection channel for entrance of a fluid under test. The electrodes and the reagent layer are in the channel. The upper cover 9 is provided with an air hole 91, which is located above the open groove and used for discharging air in the sample injection channel after addition of the sample to be tested. A color layer 7 is printed with a color or symbols for differentiating different types of sensors and is tightly adhered to the interlayer 8.

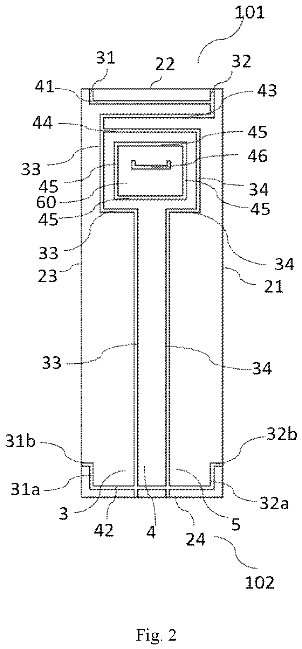

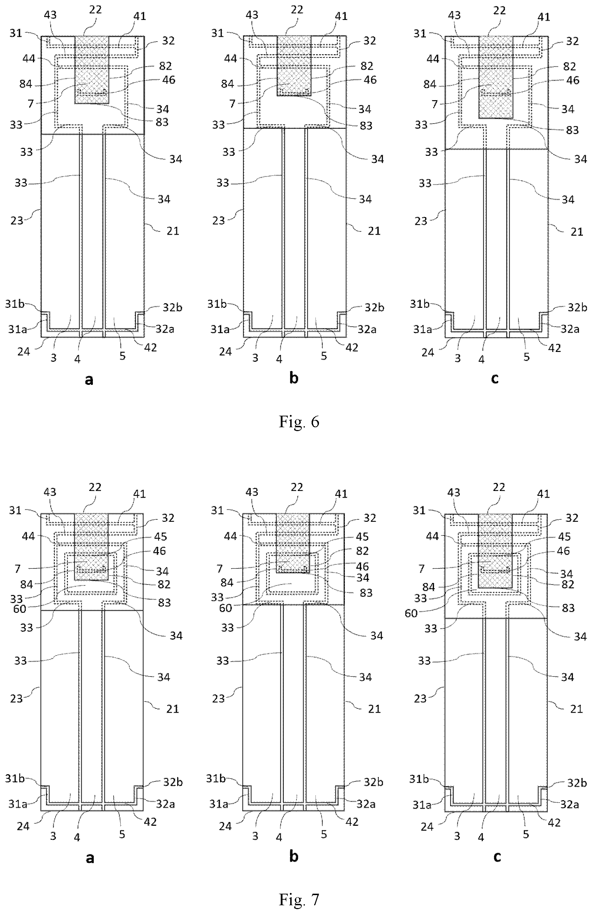

Fig. 2 is a front view of the conductive layer 2 in Fig. 1, the engraved lines are insulating gaps left by the removal of a conductive material from the conductive layer 2, and the engraved lines are not limited to gaps formed by means of engraving. As shown in Fig. 2, the working electrode 5 is enclosed by the engraved lines 32, 33, 34, 42, 43, 44, 32a, 32b, a side edge 21 and an end edge line 22, the counter electrode 4 is enclosed by the engraved lines 33, 34, 42, 44, 45, and the reference electrode 3 is enclosed by the engraved lines 31, 32, 33, 41, 42, 43, 31a, 31b, the side edge 23 and the end edge line 22. The conductive layer below the other end of the sample injection channel opposite to the sample injection port is provided with an auxiliary region 60 enclosed by the engraved line 45. In this example, the auxiliary region 60 is located with the region of the electrode 4. The transverse width of the auxiliary region 60 is greater than or equal to the width of the sample injection channel, and the projection of the lower edge 83 of the sample injection channel on the conductive layer falls within the auxiliary region. As shown in Fig. 7, a part of the auxiliary region is located within the sample injection channel.

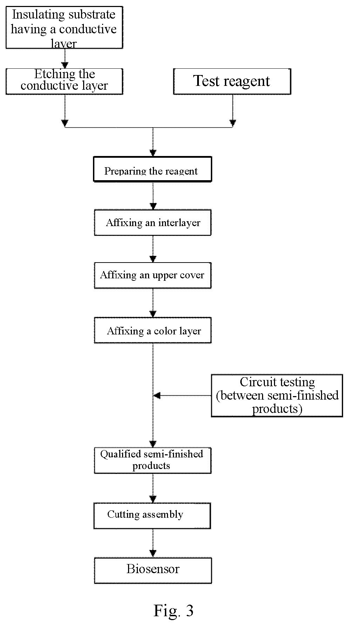

A method for manufacturing the biosensor shown in Fig. 1 is illustrated by taking Figs. 3, 4 and 5 as an example.

Step 1: laser etching of an electrode pattern: according to a pre-designed electrode pattern, on an original material having an insulating substrate and a conductive layer, etching the electrode pattern with laser on the conductive layer, thus obtaining a large electrode card with a plurality of sensor base units A.

Step 2: addition of the reagent layer: preparing a test reagent, and adding the prepared reagent to corresponding electrodes requiring addition of the reagent.

Step 3: affixing the interlayer 8 to each sensor base unit A. Generally, the interlayer is placed at the sample contacting end of the biosensor.

Step 4: sticking the upper cover 9 to each interlayer 8 and rolling.

Step 5: after the upper cover 9 is affixed, affixing and rolling the color layer 7 on the upper cover to obtain a semi-finished large card.

Step 6: cutting: cutting the semi-finished large card by using a cutter along a preset cutting line to obtain finished biosensors.

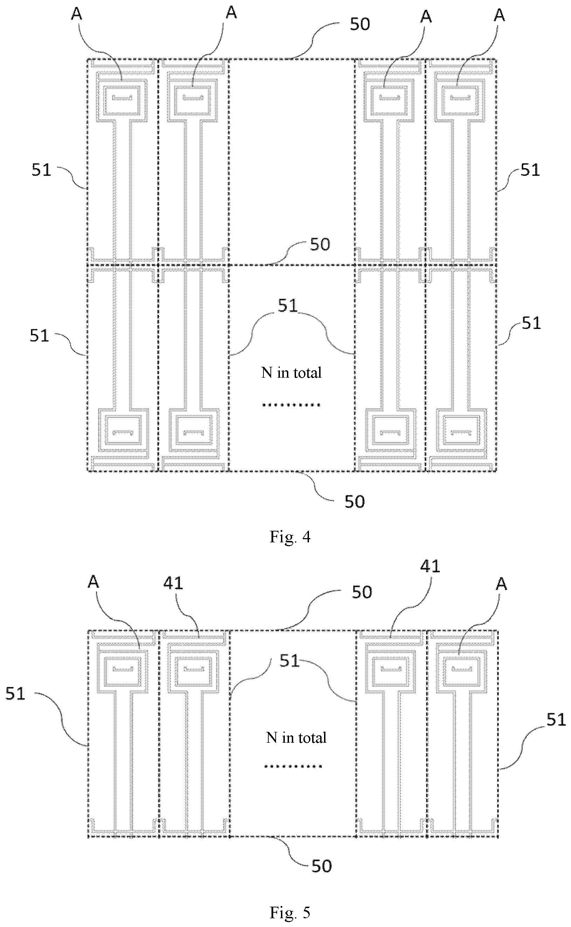

The manufacturing steps are specifically illustrated by taking Fig. 2, Fig. 4 and Fig. 5 as an example. In step 1, the conductive layer 2 on the surface of the insulating substrate is etched using the laser etching technology to form a plurality of engraved lines 31 to 34, 31a, 31b, 32a, 32b, and engraved lines 41 to 46 on the conductive layer 2, and the conductive layer in these engraved lines is basically cleared away to expose the insulating substrate. Through division by the engraved lines, the required electrodes and other engraved lines with certain uses are formed on the conductive layer. After laser etching is completed, a large electrode card is obtained, including a plurality of sensor base units A on an insulating substrate, which are arranged side by side and can be used to manufacture finished biosensors. Each sensor base unit A includes an insulating substrate, an electrode system formed after division by engraved lines, the engraved lines, and other blocks formed by etching. In this example, the electrode system includes the working electrode 5, the counter electrode 4, and the reference electrode 3. As shown in Fig. 4, two rows of sensor base units A arranged in sequence tail to tail are formed after etching (the contact ends of two sensor base units A are opposed to each other up and down), wherein N represents 0 to N; and "N in total" represents inclusion of N sensor base units A.

The engraved lines described in the present invention are not only straight lines, but also can be arcs, wavy lines and other curves. The transverse engraved line does not mean that it needs to be parallel to the two end edges of the sample end and contact end of the biosensor, and the transverse engraved line may be parallel to the end edges or at an angle to the end edges. The longitudinal engraved line also does not mean that it needs to be parallel to two side edges of the biosensor, and the longitudinal engraved line may be parallel to the side edges or at an angle to the side edges. The transverse engraved line or the longitudinal engraved line can be a straight line, a wavy line or in other shape.

In step 1, i.e., laser etching of an electrode pattern, the etching travel line of the engraved lines within the conductive layer is controlled by software. Taking Fig. 4 as an example, the engraved lines 32, 44 and 33 are formed in such a way that they are obtained from continuous laser cutting. The specific implementation of the path and direction and the like of the engraved lines can be artificially designed and controlled by software according to the needs of actual products, and is not limited to the above path. Suitable laser etching parameters are used for the engraved lines, e.g., the width of the engraved lines is within the range of 0.020mm to 0.200mm, and the width of the engraved lines used in this example is 0.080mm.

Fig. 4 is just one of the forms formed after step 1, and there may be other forms of arrangements as well, as long as the sensor base units A can be effectively cut in the back-end process. It is also possible to use Fig. 4 as a unit, and the large electrode card formed after etching is an arrangement of a plurality of units shown in Fig. 4, for example, if Fig. 4 is used as a repeating unit, N is 4 and the times of repetition is 2, the manufactured semi-finished product may have four rows and four columns.

The material of the insulating substrate is polyvinyl chloride, polyterephthalic acid, polyethylene terephthalate, macrogol ester, etc., with polyethylene terephthalate being preferred in this example.

The material of the conductive layer 2 is selected from, but not limited to conductive metals or conductive non-metals such as gold, silver, platinum, palladium, carbon, graphite, conductive glass, or a mixture thereof. The method for covering the conductive layer may use printing, coating, electroplating, sputtering, etc., and this example uses a conductive carbon layer (carbon film) formed in a coating manner, the thickness of the carbon layer is 1-30um, the thickness used in this example is about 8um, and the resistance of the conductive layer is 10 Ω/□ to 100 Ω/□, preferably 30 Ω/□ in this example.

Some or all of the electrodes of the biosensor may be configured with a reagent layer. The reagent layer employs a specific enzyme to quantitatively or qualitatively analyze the measured target substance under the environment of a buffer system, generally containing the following components: enzyme, electronic mediator, high polymer, disintegrant, surfactant, stabilizer and buffer system. Depending on the needs of testing items, the enzyme is selected from one or more of glucose oxidase, glucose dehydrogenase, lactate oxidase, lactate dehydrogenase, urate oxidase, cholesterol oxidase or D-3-hydroxybutyrate oxidase, etc.; the electronic mediator is selected from one or more of ruthenium compounds, potassium ferricyanide, ferrocene, etc.; the buffer system is any one or more of sodium succinate, sodium citrate, piperazine buffer, propanesulfonic acid, PBS buffer or sodium fumarate; the disintegrant is any one or more of crosslinked PVP, sodium carboxymethyl starch, or crosslinked CCNa; the surfactant is any one or more of anionic surfactant, cationic surfactant, diatomic surfactant, or nonionic surfactant; and the stabilizer is one or more of maltitol, trehalose, BSA, or protein protective agent. The above components after preparation are stirred sufficiently, so that the components are fully dissolved and dispersed to form a homogeneous solution. In this example, the biosensor as shown in Fig. 1 includes a reagent layer 6.

In step 2 for preparing the reaction reagent, methods such as dispensing, screen printing, drop coating and Slot-Die coating are usually adopted to configure a reagent mixture having certain chemical components onto a specific electrode of the biosensor to form the reagent layer. It is preferred in this example that screen printing is used to configure a biochemical reagent on the specific electrode in specific pattern and position. The biochemical reagent in the specific pattern and position can react with the substance to be tested in the sample and generate a certain electrical signal, and the electrical signal is conducted to a test instrument and fed back to a user via a conductive material. The screen printing plate used in the screen printing generally employs polyester, nylon, stainless steel and other materials, and is generally of 250 meshes to 420 meshes, the used wire screen has a wire diameter of 27um to 120um generally, and bears a maximum tension of 22 to 38N/cm generally. It is preferred in this example that a 305-mesh nylon screen printing plate with a wire diameter of 34um is used, which can bear a maximum tension of 33N/cm.

The reagent with fixed pattern and position obtained via screen printing as described above comes into contact with the sample in the sample injection channel and reacts with the substance to be tested in the sample. The sample injection channel is formed between the open groove 81 and the upper cover 9, and the surface of the upper cover facing the sample injection channel is a hydrophilic layer material. Specifically, the material of the interlayer is typically a PET substrate coated with an acrylic resin system as an adhesive material. The thickness of the interlayer is typically 75um to 150um, and the width of the open groove is typically 0.7mm to 1.8mm. In this example, the thickness of the interlayer is preferably 100um, and the width of the open groove is preferably 1.2mm.

An air hole 91 is left in the upper cover 9 at the tail of the sample injection channel, and the air hole may be round, square, rectangular, linear, or in other shapes. It is preferred in this example that the air hole is rectangular. In this example, the hydrophilic material is preferably 9901P made by 3M. The air hole of the hydrophilic material can ensure that the original air in a chamber is smoothly discharged when a blood sample flows into the sample injection channel, so as to ensure that the sample can smoothly flow into the chamber.

The color layer 7 is generally a printable single-sided adhesive tape, with the product name generally printed on the surface, which plays a role of easily identifying the product and protecting the biosensor from damage caused by scratching.

Step 5 is a step that can be omitted. For example, if the upper cover itself can be printed with product name, which can simultaneously serve a similar function as the color layer, step 5 is omitted. Alternatively, if the biosensor does not require the color layer, step 5 is omitted.

In the cutting step 6, the semi-finished large card is cut to obtain finished biosensors by using a cutter along a preset cutting line, specifically, using the cutter along the cutting lines at positions shown by transverse dotted lines 50 and longitudinal dotted lines 51 in Fig. 4. Suitable cutting methods include but not limited to hobbing, die punching, chopping, etc. Specifically, it is preferred in this example to use a hob to hob the semi-finished large card as shown in Fig. 4 along the transverse dotted line 50 into semi-finished strip-shaped sheets containing several sensor base units A as shown in Fig. 5, and then use the hob to cut the semi-finished strip-shaped sheets into finished biosensors along the vertical dotted line 51. In order to more visually illustrate the cutting position of the hob, when Fig. 4 and Fig. 5 are used to introduce the cutting process of step 6, the interlayer, the upper cover and the color layer are not shown in Fig. 4 and Fig. 5. The cutting process described in step 6 is one of the important steps in the assembly of the sensor, the effect of which directly affects the pass rate of the final finished sensors and the finished product effect of the sensors. The dotted lines labeled in Fig. 4 and Fig. 5 do not exist in the actual product processing, the same below. After cutting, the positions of the dotted lines 50 and 51 are edge lines of the biosensor, or can also be referred to as edges of the biosensor or side edges of the biosensor.

After a series of the processes above, independent sensors can be obtained. After the quality inspector completes relevant quality inspection works, the scrapped and defective products are removed through screening, and the obtained finished biosensors will enter the next stage, i.e., the packaging and shipping stage.

The quality inspection may include the content of determining the sample addition position on the reagent layer, and when the allocated position of the reagent layer exceeds a preset range, the biosensor will be detected by the quality inspector and then determined as a scrapped sensor. Specifically, when the allocated position of the reagent layer is deviated downward to result in that the engraved line 41 is not covered by the reagent layer, or the allocated position of the reagent layer is deviated upward to result in that the engraved line 45 is not covered by the reagent layer, the sensor may be considered to be a scrapped sensor out of the preset range.

Figs. 6 and 7 are described below in detail as comparative examples. Fig. 6 does not contain the auxiliary region described in the present invention, and Fig. 7 contains the auxiliary region described in the present invention.
a, b and c in Fig. 6 are front schematic diagrams of a biosensor in three different situations, i.e., after laser etching, printing of the reagent layer, and affixing of the interlayer, in which the laser-etched engraved lines covered under the reagent layer and the interlayer are indicated by dotted lines, and the reagent layer covered in the open groove of the interlayer and on the electrodes is indicated by grid-shaped filling, and at the same time, for the sake of illustration, the upper cover and the color layer are not shown in the figure, the same hereinafter. a in Fig. 6 is a front schematic diagram of the sensor with the affixed position of the interlayer meeting the requirements, and an auxiliary region 60 enclosed by the engraved line 45 is not provided in the electrode design on the conductive layer compared to the electrode design in Fig. 2. The affixed position of the interlayer satisfies the preset range of the designer, i.e. the relative positions of the edge 83 of the open groove of the interlayer and the preset label 46 of the designer are normal and not spaced too big or too small, at which point the working electrode 5 is enclosed by the engraved lines 32, 33, 34, 42, 43, 44, 32a, 32b and the side edge line 21 and the end edge line 22, the counter electrode 4 is enclosed by the engraved lines 33, 34, 42, 44, and the reference electrode 3 is enclosed by the engraved lines 31, 32, 33, 41, 42, 43, 31a, 31b and the side edge line 23 and the end edge line 22. The end lines 82, 83, 84 of the open groove of the interlayer and the end edge line 22 of the biosensor jointly form a sample injection channel for the sample, and all electrodes within the sample injection channel are covered with a reagent layer. After the test sample enters the sample injection channel and is fully immersed, the effective electrode area of the electrode 3 participating in the signal testing of the test sample at this point is enclosed by the engraved lines 41 , 43 and the edge lines 82, 84 of the open groove of the interlayer, while the effective electrode area of the electrode 5 is enclosed by the engraved lines 43, 44 and the edge lines 82, 84 of the open groove of the interlayer, while the effective area of the electrode 4 is enclosed by the engraved line 44 and the edge lines 82, 83, 84 of the open groove of the interlayer.

b in Fig. 6 and c in Fig. 6 are front schematic diagrams of two types of biosensors obtained after deviation of the affixing of the interlayer. b in Fig. 6 is a case where the affixing of the interlayer is deviated upward (deviated upward means that the edge line 83 of the open groove of the interlayer is closer to the sample injection port compared to the edge line of the open groove in a in Fig. 6), and c in Fig. 6 is a case where the affixing of the reagent layer is deviated downward (deviated downward means that the edge line 83 of the open groove of the interlayer is further away from the sample injection port). It can be found through comparison of a, b and c in Fig. 6 that the up and down deviations of the affixing of the interlayer do not result in a change of the effective electrode areas of the electrode 3 and the electrode 5; however, the effective area of the electrode 4, which comes into contact with the reagent layer in the sample injection channel and participates in the testing of the sample to be tested, changes as a result of the change of the affixed position of the interlayer. As shown in b in Fig. 6, when the affixing of the interlayer is deviated upward, the effective area of the electrode 4 (i.e., the region in b in Fig. 6 that is enclosed by the engraved line 44 and the edge lines 82, 83, 84 of the open groove of the interlayer), which comes into contact with the reagent layer in the sample injection channel and participates in the testing of the sample, is smaller than the effective area of the electrode 4 in a in Fig. 6 (i.e., the region in a in Fig. 6 that is enclosed by the engraved line 44 and the edge lines 82, 83, 84 of the open groove of the interlayer). As shown in c in Fig. 6, when the affixing of the interlayer is deviated downward, the effective area of the electrode 4 in c in Fig. 6 (i.e., the region in c in Fig. 6 that is enclosed by the engraved line 44 and the edge lines 82, 83, 84 of the open groove of the interlayer) is the same as the effective area of the electrode 4 in a in a of Fig. 6. Based on such a situation, a deviation of the test signal may be caused between different sensors due to the problem that the effective area of the electrode is changed by the deviation of the affixing of the interlayer, which may further lead to a deviation of the test result, thereby affecting the accuracy of the sensor.

Fig. 7 shows front schematic diagrams of the sensor in Figs. 1 and 2 after laser etching, printing of the reagent layer, and affixing of the interlayer, in which the laser-etched engraved lines covered under the reagent layer and the interlayer are indicated by dotted lines, the reagent layer is indicated by grid-shaped filling blocks, and at the same time, for the sake of illustration, the upper cover and the color layer are not shown in the figure. a in Fig. 7 shows a front schematic diagram of a sensor with the affixed position of the interlayer meeting requirements, and an auxiliary region 60 enclosed by the engraved line 45 is added in the electrode design on the conductive layer compared to the electrode design in Fig. 6, and the auxiliary region is located at the most distal end of the sample injection channel, and is at least partially covered under the open groove. At this point, the working electrode 5 is enclosed by the engraved lines 32, 33, 34, 42, 43, 44, 32a, 32b, the side edge line 21 and the end edge line 22, the counter electrode 4 is enclosed by the engraved lines 33, 34, 42, 44, 45, and the reference electrode 3 is enclosed by the engraved lines 31, 32, 33, 41, 42, 43, 31a, 31b, the side edge line 23 and the end edge line 22. The edge lines 82, 83, 84 of the open groove of the interlayer and the edge line 22 of the biosensor jointly form the region of the sample injection channel, and all electrodes within the sample injection channel are covered with the reagent layer. After the test sample enters the sample injection channel and is fully immersed, the effective electrode area of the electrode 3 participating in the signal testing of the test sample at this point is enclosed by the engraved lines 41, 43 and the edge lines 82, 84 of the open groove of the interlayer, while the effective electrode area of the electrode 5 is enclosed by the engraved lines 43, 44 and the edge lines 82, 84 of the open groove of the interlayer, while the effective area of the electrode 4 is enclosed by the engraved lines 44, 45 and the edge lines 82, 84 of the open groove of the interlayer.

b in Fig. 7 and c in Fig. 7 are front schematic diagrams of two types of biosensors with deviation of the affixing of the interlayer, it can be seen from the figures that the effective areas of the electrodes 3, 4, 5 remain unchanged all the time even if the case of deviation of the affixing of the interlayer exists. As shown in b in Fig. 7, when the affixing of the interlayer is deviated upward, the effective area of the electrode 4 (i.e., the region in b in Fig. 7 that is enclosed by the engraved lines 44, 45 and the edge lines 82, 84 of the open groove of the interlayer), which comes into contact with the reagent layer in the sample injection channel and participates in the testing of the sample, is the same as the effective area of the electrode 4 in a in Fig. 7 (i.e., the region in a in Fig. 7 that is enclosed by the engraved lines 44, 45 and the edge lines 82, 84 of the open groove of the interlayer). As shown in c in Fig. 7, when the affixing of the interlayer is deviated downward, the effective area of the electrode 4 in c in Fig. 7 (i.e., the region in a in Fig. 7 that is enclosed by the engraved lines 44, 45 and the edge lines 82, 84 of the open groove of the interlayer) is the same as the effective area of the electrode 4 in a in Fig. 7.

In the design solution of Fig. 7, the effective electrode areas of the three electrodes 3, 4, 5 are only related to the width of the open groove of the interlayer, but are independent of the affixed position of the interlayer up and down, i.e., the up and down affixed positions of the open groove of the interlayer. By means of the auxiliary region enclosed by the engraved line 45, the consistency of the effective areas of all the electrodes within the sample injection channel that are in contact with the reagent layer can be effectively fixed, thus ensuring the stability of the product.

It should be noted that the schematic diagrams of Figs. 6 and 7 only represent a case where all electrodes in the open groove of the interlayer are covered with the reagent layer, i.e., the size of the reagent layer is the same as or greater than the size of the open groove of the interlayer, and these schematic diagrams only take the case where the two sizes are the same as an example.

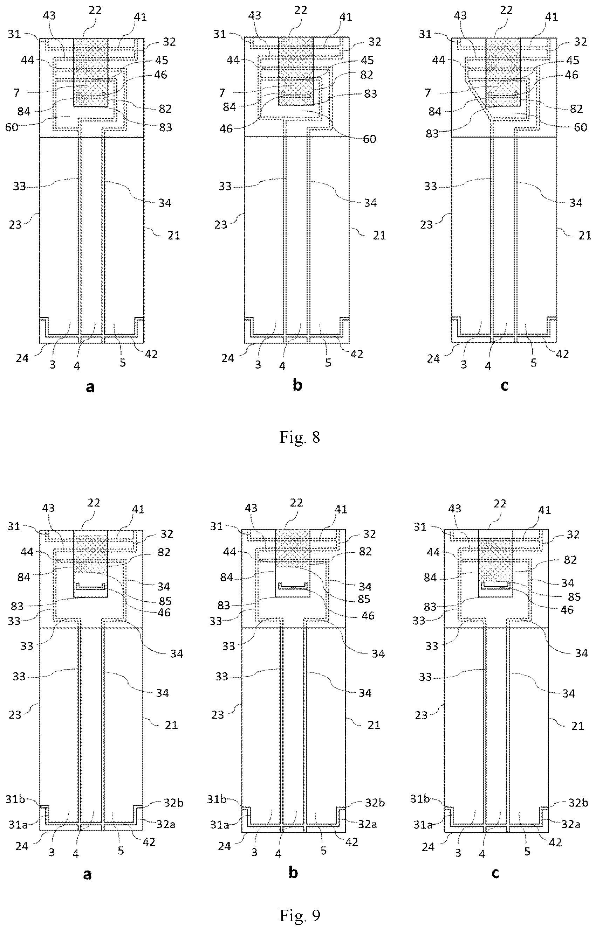

The shape and size of the auxiliary region enclosed by the engraved line as shown in Fig. 7 may vary according to the needs of the product design or the auxiliary region may be of an irregular shape, as long as it satisfies that the auxiliary region is located at the most distal end of the sample injection channel and at least is partially exposed above the lower edge of the open groove, such as auxiliary regions of different shapes enclosed by the engraved line 45 in the design solution shown in Fig. 8.

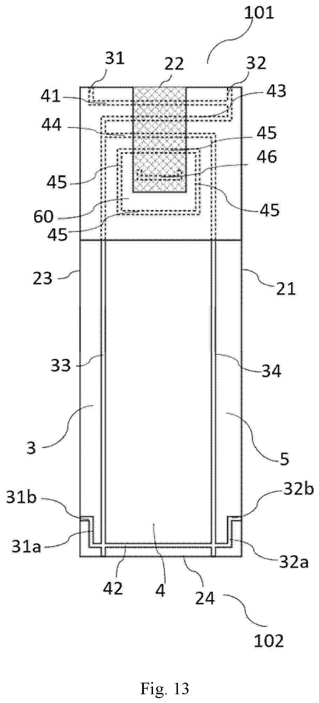

The auxiliary region 60 may be a closed area enclosed by the engraved line 45, as shown in Fig. 2 and in Fig. 13. The biosensor as shown in Fig. 13 includes a working electrode 5, a counter electrode 4 and a reference electrode. The working electrode 5 is enclosed by the engraved lines 32, 33, 34, 42, 43, 44, 32a, 32b, the side edge line 21 and the end edge line 22, the counter electrode 4 is enclosed by the engraved lines 33, 34, 42, 44, 45, the reference electrode 3 is enclosed by engraved lines 31, 32, 33, 41, 42, 43, 31a, 31b, the side edge line 23 and the end edge line 22, and the auxiliary region is enclosed by the engraved line 45.

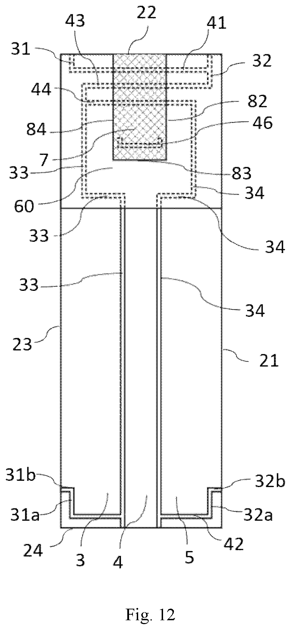

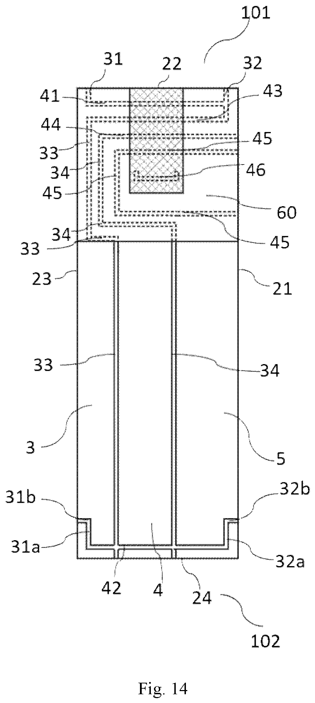

The auxiliary region 60 may also be enclosed by the engraved line 45 and an edge line of the conductive layer. As shown in Fig. 12, the auxiliary region 60 is a region enclosed by the engraved lines 33, 44, 34 and the end edge 24 of the conductive layer. As shown in Fig. 14, the biosensor includes a working electrode 5, a counter electrode 4 and a reference electrode 3. The working electrode 5 is enclosed by the engraved lines 44, 34, 42, 45 and the edge line 21, the counter electrode 4 is enclosed by the engraved lines 32, 43, 33, 42, 34, 44 and the edge lines 21, 22, the reference electrode 3 is enclosed by the engraved lines 41, 32, 42, 33, 45 and the edge lines 21 and 22, and the auxiliary region 60 is enclosed by the engraved lines 45 and the edge line 21.

As in the biosensor shown in Figs. 6 and 12, when the electrode 4 does not participate in the calculation of the test results during the testing process, for example, when the electrode 4 serves as a test electrode for determining whether the sample addition amount is sufficient or not or when the electrode 4 does not participate in any test at all, the electrode 4 likewise can also serve as an auxiliary region assisting in ensuring the effective electrode area of the electrode 3 and the electrode 5.

The auxiliary region is further away from the sample injection port than the electrodes. When the biosensor includes a working electrode and a counter electrode, the auxiliary region is further away from the sample injection port than the working electrode and the counter electrode; and when the biosensor comprise a working electrode, a counter electrode, a reference electrode and other electrode that might be required for use, the auxiliary region is further away from the sample injection port than the aforementioned electrodes.

### Example 2

The design solution of the auxiliary region provided in Example 1 of the present invention is based on the case where all electrodes in the open groove of the interlayer are allocated with a reagent layer. However, in the actual application process, the design of the auxiliary region is still applicable when the reagent does not spread over the open groove of the interlayer and only part of the electrodes are allocated with the reagent layer.

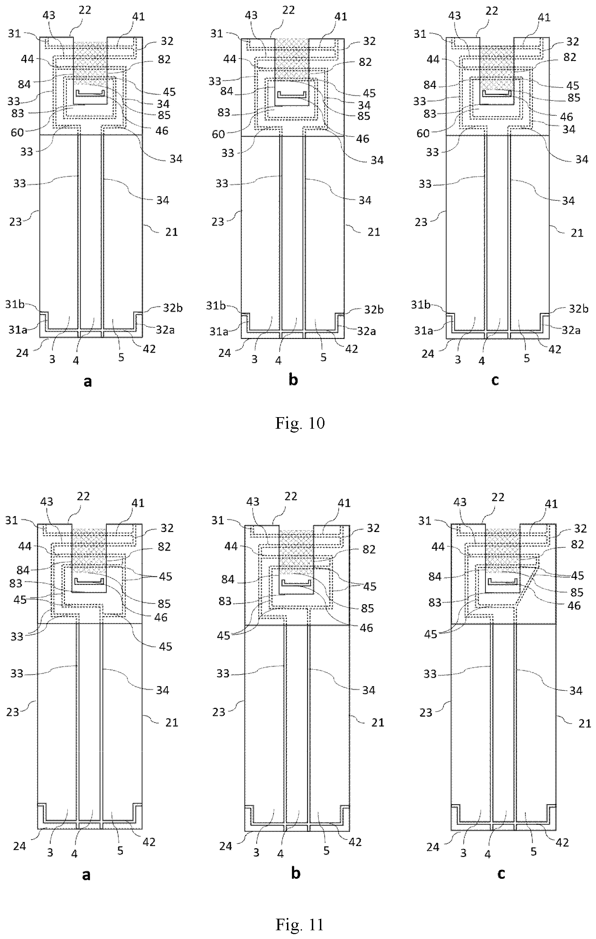

Figs. 9 and 10 are described below in detail as comparative examples.

Fig. 9 shows front schematic diagrams of a sensor after laser etching, printing of the reagent layer, and affixing of the interlayer, in which the laser-etched engraved lines covered under the reagent layer and the interlayer are indicated by dotted lines, the reagent layer covered in the open groove of the interlayer and on the electrodes is indicated by grid-shaped filling blocks, and at the same time, for the sake of illustration, the upper cover and the color layer are not shown in this schematic diagram, the same hereinafter. a in Fig. 9 shows a front schematic diagram of the sensor with the allocated position of the reagent layer meeting requirements. At this point, the working electrode 5 is enclosed by the engraved lines 32, 33, 34, 42, 43, 44, 32a, 32b, the side edge line 21 and the end edge 22, the counter electrode 4 is enclosed by the engraved lines 33, 34, 42, 44, and the reference electrode 3 is enclosed by the engraved lines 31, 32, 33, 41, 42, 43, 31a, 31b, the side edge 23 and the end edge 22. The edge lines 82, 83, 84 of the open groove of the interlayer and the edge line 22 of the biosensor jointly form the region of the sample injection channel, part of the electrodes within the sample injection channel are covered with a reagent layer, and the reagent layer is indicated by grid-shaped filling, in which the lower edge of the reagent layer is 85. After the test sample enters the sample injection channel and is fully immersed, the effective area of the electrode 3 participating in the signal testing of the test sample at this point is in the region enclosed by the engraved lines 41, 43 and the edge lines 82, 84 of the open groove of the interlayer, while the effective electrode area of the electrode 5 is in the region enclosed by the engraved lines 43, 44 and the edge lines 82, 84 of the open groove of the interlayer, while the effective area of the electrode 4 is in the region enclosed by the engraved line 44 together with the edge lines 82, 84 of the open groove of the interlayer and the lower edge line 85 of the reagent layer.

b in Fig. 9 and c in Fig. 9 are front schematic diagrams of two types of sensors obtained after deviation of the allocated position of the reagent layer. b in Fig. 9 shows a case where the allocated position of the reagent layer is deviated upward (deviated upward means that the lower edge 85 of the reagent layer is closer to the sample injection port compared to the lower edge of the reagent in a in Fig. 9), and c in Fig. 9 shows a case where the allocated position of the reagent layer is deviated downward (deviated under means that the lower edge 85 of the reagent is further away from the sample injection port compared to the lower edge 85 of the reagent in a in Fig. 9). It can be found through comparison of a, b and c in Fig. 9 that the deviation of the allocated position of the reagent layer does not result in changes of the effective electrode areas of the electrode 3 and the electrode 5; however, the effective area of the electrode 4, which comes into contact with the reagent layer in the sample injection channel and participates in the testing of the sample to be tested changes as a result of the change of the allocated position of the reagent layer. When the allocated position of the reagent layer as shown in b in Fig. 9 is deviated upward, the effective area of the electrode 4, which comes into contact with the reagent layer in the sample injection channel and participates in the testing of the sample (i.e., the region in b in Fig. 9 that is enclosed by the engraved line 44 together with the edge lines 82, 84 of the open groove of the interlayer and the lower edge line 85 of the reagent layer) is smaller than the effective area of the electrode 4 in a in Fig. 9. When the allocated position of the reagent layer in c in Fig. 9 is deviated downward, the effective area of the electrode 4 in c in Fig. 9 (i.e., the region in c in Fig. 9 that is enclosed by the engraved line 44 together with the edge lines 82, 84 of the open groove of the interlayer and the lower edge line 85 of the reagent layer) is greater than the effective area of the electrode 4 in a in Fig. 9. Based on such a situation, a deviation of the test signal may be caused between different sensors due to the problem that the effective area of the electrode is changed by the deviation of the allocated position of the reagent layer, which may further lead to a deviation of the test result, thereby affecting the accuracy of the sensor.

The problem encountered in the design solution of Fig. 9 can be effectively avoided if an auxiliary region is disposed corresponding to a tail end region of the open groove of the interlayer. Fig. 10 shows front schematic diagrams of a sensor after laser etching, printing of the reagent layer, and affixing of the interlayer, in which the laser-etched engraved lines covered under the reagent layer and the interlayer are indicated by dotted lines, the reagent layer is indicated by grid-shaped filling blocks, and at the same time, for the sake of illustration, the upper cover and the color layer are not shown in the figure. a in Fig. 10 shows a front schematic diagram of a sensor with the allocated position of the reagent layer meeting requirements, and b in Fig. 10 and c in Fig. 10 are front schematic diagrams of a sensor with the allocated positions of the reagent layer being deviated upward and downward, respectively, and with the deviation sizes not exceeding the expectation, an auxiliary region enclosed by the engraved line 45 is added in the electrode design on the conductive layer compared to the electrode design in Fig. 9, and the auxiliary region is located at the most distal end of the sample injection channel, and is at least partially covered under the reagent layer. At this point, the working electrode 5 is enclosed by the engraved lines 32, 33, 34, 42, 43, 44, 32a, 32b, the edge line 21 and the end edge 22, the counter electrode 4 is enclosed by the engraved lines 33, 34, 42, 44, 45, and the reference electrode 3 is enclosed by the engraved lines 31, 32, 33, 41, 42, 43, 31a, 31b, the edge line 23 and the end edge 22. The edge lines 82, 83, 84 of the open groove of the interlayer and the edge line 22 of the biosensor jointly form the region of the sample injection channel, and a part of the electrodes within the sample injection channel are covered with a reagent layer, of which the lower edge is indicated by 85. After the test sample enters the sample injection channel and is fully immersed, the effective area of the electrode 3 participating in the signal testing of the test sample at this point is enclosed by the engraved lines 41, 43 and the edge lines 82, 84 of the open groove of the interlayer, while the effective electrode area of the electrode 5 is enclosed by the engraved lines 43, 44 and the edge lines 82, 84 of the open groove of the interlayer, while the effective area of the electrode 4 is enclosed by the engraved lines 44, 45 and the edge lines 82, 84 of the open groove of the interlayer.

b and c in Fig. 10 are cases where the allocation of the reagent layer is deviated upward or downward, and it can be seen from the figures that the effective areas of the electrodes 3, 4, 5 are not changed all the time even if the situation that the allocated position of the reagent layer is deviated upward or downward exists.

b in Fig. 10 shows a case where the allocation of the reagent layer is deviated upward, the effective area of the electrode 4, which comes into contact with the reagent layer in the sample injection channel and participates in the testing of the sample (i.e., the region in b in Fig. 10 that is enclosed by the engraved lines 44, 45 together with the edge lines 82, 84 of the open groove of the interlayer) is the same as the effective area of the electrode 4 in a in Fig. 10 (i.e., the region in a in Fig. 10 that is enclosed by the engraved lines 44, 45 and the edge lines 82, 84 of the open groove of the interlayer). c in Fig. 10 shows a case where the allocation of the reagent layer is deviated downward, the effective area of the electrode 4 in c in Fig. 10 (i.e., the region in c in Fig. 10 that is enclosed by the engraved lines 44, 45 together with the edge lines 82, 84 of the open groove of the interlayer) is greater than the effective area of the electrode 4 in a in Fig. 10.

In the design solution of Fig. 10, the effective electrode areas of the three electrodes 3, 4, 5 are only related to the width of the open groove of the interlayer, but are independent of the allocated position of the reagent layer. By means of the auxiliary region enclosed by the engraved line 45, the consistency of the effective areas of all the electrodes within the sample injection channel that are in contact with the reagent layer can be effectively fixed, thus ensuring the stability of the product.

The shape and size of the auxiliary region enclosed by the engraved lines may vary according to the needs of the product design, or the auxiliary region may be of an irregular shape, for example, as shown in a of Fig. 11, the auxiliary region consists of a 7-shaped region enclosed by the engraved lines 45 and 34; as shown in Fig. 11b, the auxiliary region consists of a rectangle with a larger area enclosed by the engraved line 45; and as shown in c in Fig. 11, the auxiliary region consists of a right-angled trapezium enclosed by the engraved line 45.

## Claims

1. A biosensor, comprising an insulating substrate, a sample injection port, a sample injection channel for entrance of a test sample, and a conductive layer disposed on the insulating substrate, engraved lines and electrodes divided by the engraved lines being distributed on the conductive layer, a reagent layer being disposed on part or all of the electrodes located in the region of the sample injection channel, wherein an auxiliary region enclosed by an engraved line or by an engraved line and an edge line of the biosensor is disposed on the conductive layer under the other end of the sample injection channel opposite to the sample injection port, the transverse width of the auxiliary region is greater than or equal to the width of the sample injection channel, and the projection of the lower edge of the sample injection channel on the conductive layer falls within the auxiliary region or on the border of the auxiliary region.

2. The biosensor according to claim 1, wherein the auxiliary region is located at the most distal end of the sample injection channel.

3. The biosensor according to claim 1, wherein the auxiliary region is further away from the sample injection port than the electrodes.

4. The biosensor according to claim 1, wherein the electrodes comprise a working electrode and a counter electrode, and the auxiliary region is further away from the sample injection port than the working electrode and the counter electrode; or the electrodes comprise a working electrode, a counter electrode and a reference electrode, and the auxiliary region is further away from the sample injection port than the working electrode, the counter electrode and the reference electrode.

5. The biosensor according to claim 1, wherein a part of the auxiliary region is located in the sample injection channel.

6. The biosensor according to claim 1, wherein the lower edge of the reagent layer in the sample injection channel is located within the auxiliary region.

7. A method for preparing a biosensor, comprising the following steps: on an original material having an insulating substrate and a conductive layer, etching the conductive layer to form engraved lines and electrodes divided by the engraved lines; adding a prepared reagent to corresponding electrodes; and sticking an interlayer and an upper cover in sequence on the electrodes, wherein an auxiliary region enclosed by an engraved line or by an engraved line and an edge line of the biosensor is disposed on the conductive layer under the other end of the sample injection channel opposite to the sample injection port, the transverse width of the auxiliary region is greater than or equal to the width of the sample injection channel, and the projection of the lower edge of the sample injection channel on the conductive layer falls within the auxiliary region or on the border of the auxiliary region.

8. The method according to claim 7, wherein the auxiliary region is located at the most distal end of the sample injection channel.

9. The method according to claim 7, wherein the auxiliary region is further away from the sample injection port than the electrodes.

10. The method according to claim 7, wherein the etching method is laser cutting or laser ablation.
